# EUROPEAN PATENT APPLICATION

(11) **EP 4 813 281 A1**
(43) Date of publication of application: **30.09.2026**
(21) Application number: 23956468.5
(22) Date of filing: 26.10.2023
(51) Int. Cl.: A61B 1/00, A61B 1/012, A61B 1/018, A61B 1/015, A61B 1/05

(54) **ENDOSCOPE AND ENDOSCOPE INSERTION TUBE ASSEMBLY**

(71) Applicant: Macrolux Medical Technology Co., Ltd., Shenzhen, Guangdong 518132 (CN)
(72) Inventor: PAN, Hanzhi, Shenzhen, Guangdong 518132 (CN); WANG, Jin, Shenzhen, Guangdong 518132 (CN)
(74) Representative: Metida
(86) International application number: PCT/CN2023/126921
(87) International publication number: WO 2025/086220

(57) **Abstract**

The present utility model relates to an endoscope and an endoscope insertion tube assembly. The insertion tube assembly includes a main-body segment, an open-port segment, a tip segment, an imaging module, and an instrument channel tube. A first channel is arranged in the main-body segment. The open-port segment is connected to a distal end of the main-body segment. A second channel and a third channel isolated from each other are arranged in the open-port segment, and at least a portion of the second channel and the third channel are arranged side by side in a radial direction of the open-port segment. An instrument outlet communicating with the second channel is arranged on an outer peripheral surface of the open-port segment. The third channel communicates with the first channel. A fourth channel is arranged in the tip segment, and a through-flow port is arranged on a distal end surface of the tip segment. The utility model enables a smaller insertion tube diameter while maintaining a large instrument channel, irrigation channel, and reflux channel.

## Description

### TECHNICAL FIELD

The present utility model relates to an endoscope and an insertion tube assembly of the endoscope.

### BACKGROUND

With the rapid development of science and technology and medical technologies, minimally invasive or non-invasive medical examination and/or treatment using an endoscope has become widely used. An endoscope generally includes an insertion tube assembly, a handle assembly, and a connecting cable. Generally, an outer diameter of the insertion tube assembly of the endoscope is made as small as possible, so that, when medical personnel operate an insertion tube of the endoscope to pass through a natural body cavity or a minimally invasive surgical channel to a specific position, discomfort of a patient can be reduced and compliance of the patient can be improved. After the endoscope reaches the specific position, tissue can be observed. When abnormal tissue is observed, it is often necessary to insert an instrument, such as biopsy forceps or surgical scissors, to the specific position through an instrument channel in the endoscope to take a biopsy, or to insert a treatment device to treat diseased tissue. In this case, a larger inner diameter of the instrument channel allows instruments with larger outer diameters and more types to pass therethrough, and provides more options for performing biopsy and delivering treatment.

In addition, when an endoscope enters a natural body cavity, the cavity is often in a collapsed state. In this case, the endoscope is often further required to be provided with a fluid inflow/outflow assembly, so that liquid can be used to fill and expand the collapsed cavity to a distended state through a tube in the endoscope, thereby preventing a head end of the endoscope from scraping mucosa of the natural body cavity or even contusing the cavity. In addition, only after the internal cavity and organs are expanded can a camera clearly observe diseased tissue without obstruction. In this case, an irrigation (inflow) channel of the endoscope is required to have a sufficiently large cross-sectional area to ensure a sufficient liquid flow rate for expanding the cavity. Meanwhile, because the expansion pressure bearable by human tissue is limited, the endoscope is further required to have a reflux (water outlet) channel. Particularly when the endoscope is applied to examination and treatment of a female uterus, the endoscope needs to pass through a narrow cervical opening. After entering the uterus, the endoscope is required to quickly distend the uterus, and only after uterine distension can the uterus be clearly observed. After a clear field of view is obtained due to uterine distension, a doctor further needs to observe the whole uterus by moving an endoscope body forward and backward, swinging the endoscope body, or rotating the endoscope body. When abnormal tissue is found, biopsy forceps, scissors, or another instrument for biopsy or treatment is then inserted through the instrument channel of the endoscope. Therefore, when an endoscope is applied to special scenarios such as examination and treatment of a female uterus, the insertion tube of the endoscope is required to have a smaller outer diameter, the instrument channel is required to have a larger inner diameter, and an irrigation (inflow) channel and a reflux (water outlet) channel are required to implement rapid uterine distension.

In summary, current overall requirements for the physical dimensions of an endoscope are as follows: a smaller outer diameter of the insertion tube assembly, a larger inner diameter of the instrument channel, a large irrigation (inflow) channel, and a large reflux (water outlet) channel. At present, optical and electronic technologies have become highly advanced, and dimensions of an imaging component and an illuminating component are basically fixed. Therefore, enabling the insertion tube assembly to have a small diameter while ensuring the large inner diameter of the instrument channel , the large irrigation (inflow) channel, and the large reflux (water outlet) channel is of great significance for patients, doctors, and endoscope manufacturers.

### SUMMARY

### Technical Problem

The present utility model mainly solves the problem of how to enable an insertion tube assembly to have a small diameter while ensuring a large instrument channel inner diameter, a large irrigation (inflow) channel, and a large reflux (water outlet) channel.

### Technical Solution

According to a first aspect, the present utility model provides an insertion tube assembly for an endoscope.

An insertion tube assembly for an endoscope includes:
a main-body segment, configured to be connected to a distal end of a handle assembly of the endoscope, a first channel being arranged in the main-body segment;
an open-port segment, connected to a distal end of the main-body segment; a second channel and a third channel being arranged in the open-port segment, the second channel and the third channel being isolated from each other, at least a portion of the second channel and the third channel being arranged side by side in a radial direction of the open-port segment; an instrument outlet communicating with the second channel being arranged on an outer peripheral surface of the open-port segment, and the third channel communicating with the first channel;
a tip segment, connected to a distal end of the open-port segment; a fourth channel being arranged in the tip segment, a through-flow port communicating with the fourth channel being arranged on a distal end surface of the tip segment, and a proximal end of the fourth channel communicating with the third channel;
an imaging module, mounted on the tip segment;
and an instrument channel tube, arranged in the first channel, a distal end of the instrument channel tube being hermetically connected to a proximal end of the second channel, and a proximal end of the instrument channel tube being configured to be connected to the handle assembly of the endoscope.

In one technical solution, a distal end of the second channel is a groove structure, the groove structure forms the instrument outlet, a groove wall of the groove structure forms a guiding slope surface, and the guiding slope surface is configured to guide an instrument out of the insertion tube assembly and prevent the instrument from swinging toward either side in a width direction of the groove structure; the second channel and the third channel have a common partition layer, and the guiding slope surface is arranged on the partition layer.

In one technical solution, the fourth channel is offset toward one radial side of the tip segment, an offset direction is consistent with a direction in which the instrument outlet faces, and a proximal opening of the fourth channel faces a distal side of the partition layer.

In one technical solution, the groove structure includes a straight portion and an arcuate portion, the straight portion is located on a proximal side of the instrument outlet and extends in an axial direction of the open-port segment, and the arcuate portion is connected to a distal end of the straight portion.

In one technical solution, a guiding groove is arranged on an outer peripheral surface of the tip segment, the guiding groove is butted with a distal end of the groove structure surface, and a groove wall of the guiding groove forms an extended guiding surface configured to guide bending of the instrument.

In one technical solution, the guiding groove penetrates through the tip segment.

In one technical solution, a cross section on a side of the second channel close to the third channel is arcuate, causing wedge-shaped spaces to be formed at two circumferential ends of the third channel.

In one technical solution, the distal end surface of the tip segment has an inclined portion, the inclined portion is located on at least one circumferential side of the distal end surface, and at least a portion of the through-flow port is located on the inclined portion.

In one technical solution, the fourth channel is located on one radial side of the tip segment, and the imaging module is located on another radial side of the tip segment; a cross section of the fourth channel is substantially an isosceles trapezoid, a first base of the isosceles trapezoid is close to the imaging module, and a second base of the isosceles trapezoid is away from the imaging module.

In one technical solution, two ends of the first base have recesses, and the recesses are recessed toward a side of the tip segment on which the imaging module is arranged.

In one technical solution, the insertion tube assembly further includes an illuminating module, and the illuminating module is arranged on two sides of the imaging module along an extension direction of a base of the isosceles trapezoid.

In one technical solution, the main-body segment, the open-port segment, and an end-port segment are three components connected in sequence, and the open-port segment is manufactured by an injection molding, powder metallurgy, ceramic sintering, or 3D printing process.

In one technical solution, the imaging module is connected to a connecting wire, the connecting wire passes through the third channel and the first channel, and a gap for liquid flow is present between the connecting wire and inner walls of the third channel and the first channel.

According to a first aspect, the present utility model provides an endoscope.

An endoscope includes:
an insertion tube assembly, the insertion tube assembly being the insertion tube assembly according to any one of the foregoing technical solutions;
and a handle assembly, a proximal end of the insertion tube assembly being connected to the handle assembly, an inflow port and a reflux port being arranged on the handle assembly, one of the inflow port and the reflux port communicating with the proximal end of the instrument channel tube, and the other communicating with a space in the first channel around the instrument channel tube.----

### Beneficial Effects

Beneficial effects of the present utility model are as follows.

According to the above endoscope, because the insertion tube assembly is provided with the second channel and the third channel arranged side by side in the radial direction, the second channel corresponds to the instrument outlet on the outer peripheral surface of the insertion tube assembly and is connected to the instrument channel tube, so that an instrument can pass through the second channel, the instrument channel is prevented from occupying a relatively large radial dimension, and the second channel can be used as a liquid channel to implement water inlet of the endoscope, for irrigating a cavity, or reflux, for returning liquid in the cavity. Meanwhile, the imaging module and the fourth channel are both arranged on a distal end surface of the insertion tube assembly, namely the distal end surface of the tip segment, and a flow channel communicating with the fourth channel is arranged in the insertion tube assembly on a proximal side of the imaging module, so that reflux of the endoscope, for returning liquid in the cavity, or water inlet, for irrigating the cavity, can be correspondingly implemented. As a whole, a smaller diameter is achieved while water inlet and water outlet of the endoscope are ensured.

### BRIEF DESCRIPTION OF THE DRAWINGS

FIG. 1 is a perspective view of an embodiment of an endoscope according to the present utility model.
FIG. 2 is a perspective view of the insertion tube assembly in FIG. 1.
FIG. 3 is a longitudinal sectional view of FIG. 2.
FIG. 4 is an exploded structural view of the insertion tube assembly in FIG. 2.

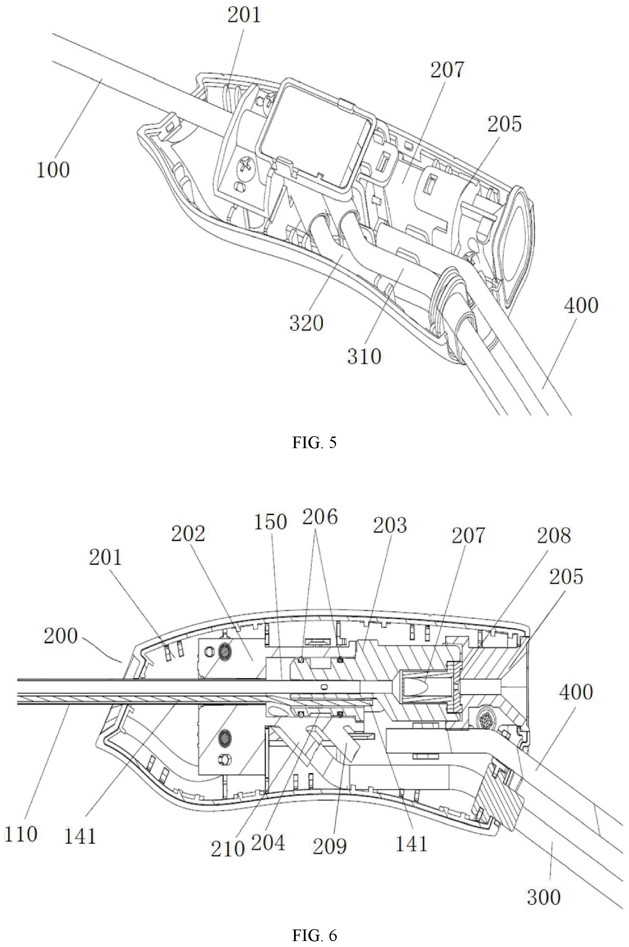

FIG. 5 is a schematic diagram showing an internal structure of the handle in FIG. 1.
FIG. 6 is a longitudinal sectional view of FIG. 5.
FIG. 7 is a perspective view of another embodiment of an endoscope according to the present utility model.
FIG. 8 is a longitudinal sectional view of the insertion tube assembly in FIG. 7.
FIG. 9 is an exploded structural view of the insertion tube assembly in FIG. 8.

A list of feature names corresponding to reference numerals in the drawings is as follows:
100 - insertion tube assembly; 110 - main-body segment; 120 - open-port segment; 121 - second channel; 122 - guiding slope surface; 123 - straight portion; 124 - arcuate portion; 125 - third channel; 126 - instrument outlet; 130 - tip segment; 131 - fourth channel; 132 - guiding groove;
133 - inclined portion; 134 - recess; 135 - through-flow port; 136 - fifth channel; 140 - imaging module; 141 - connecting wire; 150 - instrument channel tube; 160 - illuminating module;
200 - handle assembly; 201 - handle housing; 202 - insertion tube mounting seat; 203 - sealing adapter; 204 - annular groove; 205 - instrument access seal cap; 206 - O-ring; 207 - one-way duckbill valve; 208 - one-way silicone pad; 209 - inflow port; 210 - reflux port;
300 - fluid inflow/outflow assembly; 310 - infusion pipe; 320 - reflux pipe;
400 - external cable.

### DETAILED DESCRIPTIONS OF THE EMBODIMENTS

The present utility model is further described in detail below through specific implementations with reference to the accompanying drawings. Similar elements in different implementations are denoted by associated similar reference numerals. In the following implementations, many details are described to enable a better understanding of the present application. However, a person skilled in the art can readily recognize that some features may be omitted in different circumstances or may be replaced by other elements, materials, or methods. In some cases, some operations related to the present application are not shown or described in the specification, so as to avoid obscuring the core part of the present application with excessive description. For a person skilled in the art, detailed description of these related operations is unnecessary, and the person skilled in the art can fully understand the related operations based on the description in the specification and common general knowledge in the art.

In addition, the characteristics, operations, or features described in the specification may be combined in any suitable manner to form various implementations. Meanwhile, the steps or actions in the method description may also be changed or adjusted in sequence in a manner obvious to a person skilled in the art. Therefore, various sequences in the specification and drawings are merely for clearly describing one embodiment, and do not mean that the sequences are necessary unless a particular sequence is otherwise expressly stated to be followed.

The serial numbers assigned to components herein, such as "first" and "second," are used merely to distinguish described objects, and have no sequential or technical meaning. In addition, "connection" and "coupling" in the present application include direct and indirect connection (coupling) unless specifically stated otherwise.

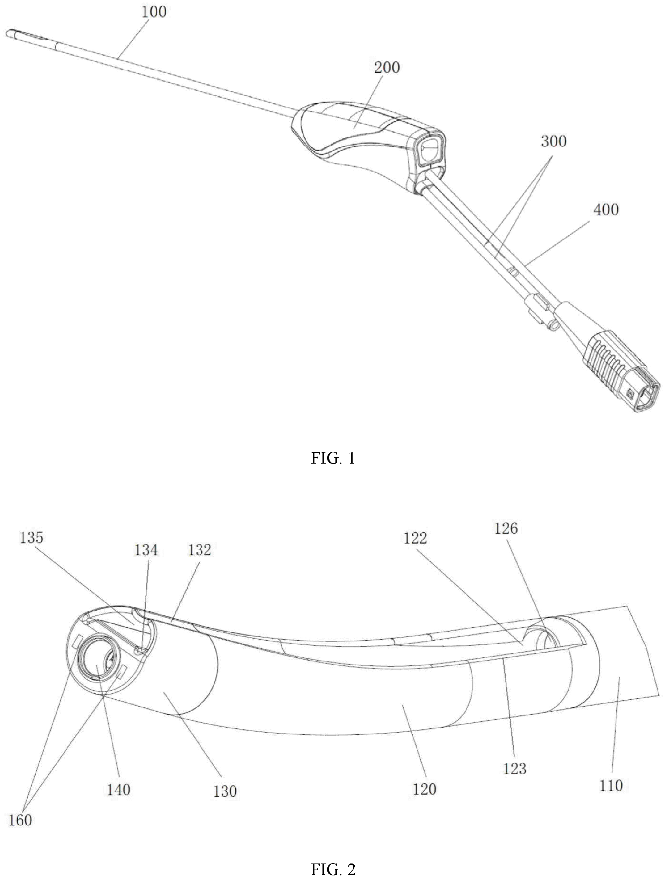

In an embodiment of the endoscope of the present utility model, the endoscope may be a hysteroscope. Referring to FIG. 1, the endoscope may include an insertion tube assembly 100, a handle assembly 200, a fluid inflow/outflow assembly 300, and an external cable 400. The insertion tube assembly 100 is configured to be inserted into a target position, observe the target position, and provide a guiding channel for a corresponding instrument. The handle assembly 200 can serve as a connection base of the insertion tube assembly 100 and can be held by an operator to perform corresponding operations. The fluid inflow/outflow assembly 300 is configured to implement filling and irrigation of a cavity. The external cable 400 is configured to transmit electricity to satisfy output of images and power supply to the illuminating module 160. Certainly, it should be noted that the endoscope in the present utility model may also be used in other scenarios besides a hysteroscope.

In one embodiment, the insertion tube assembly 100 of the endoscope includes a main-body segment 110, an open-port segment 120, and a tip segment 130. A length of the main-body segment 110 is often the longest part of the insertion tube assembly 100, and the main-body segment 110 is configured to be connected to a distal end of the handle assembly 200 of the endoscope. Movement, rotation, and the like of the insertion tube assembly 100 can be implemented through movement, rotation, and the like of the handle assembly 200. In a specific embodiment, the main-body segment 110 may be a cylindrical hollow tube body, and an inner cavity thereof forms a first channel through which a corresponding instrument channel tube 150 and connecting wire 141 can pass. Specifically, the instrument channel tube 150 is arranged in the first channel, a distal end of the instrument channel tube 150 is hermetically connected to a proximal end of the second channel 121, and a proximal end of the instrument channel tube 150 is configured to be connected to a handle of the endoscope. The instrument channel tube 150 and the connecting wire 141 are described in further detail below.

The open-port segment 120 is connected to a distal end of the main-body segment 110. A second channel 121 and a third channel 125 are arranged within the open-port segment 120. The second channel 121 and the third channel 125 are isolated from each other, and the third channel 125 and at least a portion of the second channel 121 are arranged side by side in a radial direction of the open-port segment 120. The second channel 121 is an instrument channel, and is also a water inlet channel or a reflux channel. An instrument outlet 126 communicating with the second channel 121 is arranged on an outer peripheral surface of the open-port segment 120, and an instrument such as biopsy forceps or scissors inserted through the instrument channel tube 150 can extend out through the instrument outlet 126 to perform a corresponding operation. Correspondingly, the third channel 125 is a reflux channel or an inflow channel, and may also serve as a passage for the connecting wire 141 to allow a cable, connected to a corresponding module at a distal end of the insertion tube assembly 100, to be led out. The third channel 125 communicates with the first channel, so that the third channel 125 can be connected to the handle assembly 200.

In one embodiment, to facilitate processing of the open-port segment 120, the main-body segment 110, the open-port segment 120, and an end-port segment are three components connected in sequence. The open-port segment 120 is manufactured by an injection molding, powder metallurgy, ceramic sintering, or 3D printing process, and may be made of plastic, PEEK, metal, or the like. The manufacturing process is simple and low in cost, and facilitates ensuring shape and dimensional tolerances and ensuring sealing performance between the instrument channel and the irrigation channel.

To facilitate connection between the open-port segment 120 and the main-body segment 110, a proximal outer peripheral surface of the open-port segment 120 is an outer step segment having a smaller diameter than an adjacent part, and can be inserted into a distal end of the main-body segment 110. To facilitate smooth passage of an instrument, a proximal inner peripheral surface of the second channel 121 in the open-port segment 120 is an inner step segment having a larger diameter than an adjacent part, so that an inner wall of the instrument channel tube 150 can be flush with a proximal inner wall of the second channel 121 without a diameter difference.

If the connecting wire 141 passes through the third channel 125, the connecting wire 141 occupies part of the space. To avoid affecting liquid irrigation, a cross section on a side of the second channel 121 close to the third channel 125 is arcuate, causing wedge-shaped spaces to be formed at two circumferential ends of the third channel 125. The arcuate shape may be implemented by setting an outer contour of a cross section of the second channel 121 to be circular. In some possible embodiments, if a wireless imaging module 140 is used, the connecting wire 141 may be omitted.

The tip segment 130 is connected to a distal end of the open-port segment 120. A fourth channel 131 is arranged in the tip segment 130. A through-flow port 135 communicating with the fourth channel 131 is arranged on a distal end surface of the tip segment 130, and a proximal end of the fourth channel 131 communicates with the third channel 125. By arranging the tip segment 130, not only can the imaging module 140 be mounted, but irrigation or reflux can also be implemented through the through-flow port 135. In addition, one of an inflow opening and a reflux opening is arranged on the distal end surface of the insertion tube assembly 100, and the other is arranged on a side surface of the insertion tube assembly 100. This design facilitates forming a large liquid circulation for irrigation (water inlet) and reflux (water outlet), improves efficiency of uterine distension, and avoids a situation in which, when both irrigation (water inlet) and reflux (water outlet) are arranged on a head-end surface, liquid flows out from the irrigation port and is immediately discharged again. In addition, only one circulation port is arranged on the tip segment 130, so that a relatively large liquid circulation through-flow area can be achieved while the diameter of the insertion tube assembly 100 is controlled, thereby improving liquid irrigation efficiency.

It should be clarified that the instrument outlet 126 on the outer peripheral surface of the insertion tube assembly 100 may serve as an inflow opening or a reflux opening. Correspondingly, the through-flow port 135 on the distal end surface of the insertion tube assembly 100 may serve as a reflux opening or an inflow opening.

To facilitate insertion into a natural body cavity and avoid tissue or mucosa being scratched by a sharp corner, the distal end surface of the tip segment 130 has an inclined portion 133. The inclined portion 133 is located on at least one circumferential side of the distal end surface, and at least one portion of the through-flow port 135 is located on the inclined portion 133. In some other embodiments, when the imaging module 140 is located in the middle of the tip segment 130, the inclined portion 133 may also surround the tip segment 130 for one circumference to form a conical surface.

In one embodiment of the present utility model, the fourth channel 131 is located on one radial side of the tip segment 130, and the imaging module 140 is located on another radial side of the tip segment 130. Based on this, a cross section of the fourth channel 131 uses an irregular design and is substantially an isosceles trapezoid. A first base of the isosceles trapezoid is close to the imaging module 140, and a second base is away from the imaging module 140, so that the fourth channel 131 can fully adapt to a cylindrical structure of the tip segment 130 and the cross-sectional area of the fourth channel 131 can be further increased. The substantially isosceles trapezoid means that an overall contour of the cross section is approximately an isosceles trapezoid; two legs of the isosceles trapezoid may have curvature, local portions of the two bases may have protrusions and recesses, and the two bases may also have curvature.

Further, in a specific embodiment, the first base is a straight side, two ends of the first base have recesses 134, and the recesses 134 are recessed toward a side of the tip segment 130 on which the imaging module 140 is arranged. The recesses 134 may be arcuate or square. In some embodiments, a chamfer may also be formed between a base and a leg of the isosceles trapezoid. In addition, in some other embodiments, the cross-sectional shape of the fourth channel 131 may also be another shape, such as a circle, a triangle, or a semicircle.

When the illuminating module 160 needs to be arranged, the illuminating module 160 may be arranged on two sides of the imaging module 140 along the extension direction of the base of the isosceles trapezoid, so that a radial dimension of the tip segment 130 can be more effectively used and an overall outer diameter of the insertion tube assembly 100 can be better controlled.

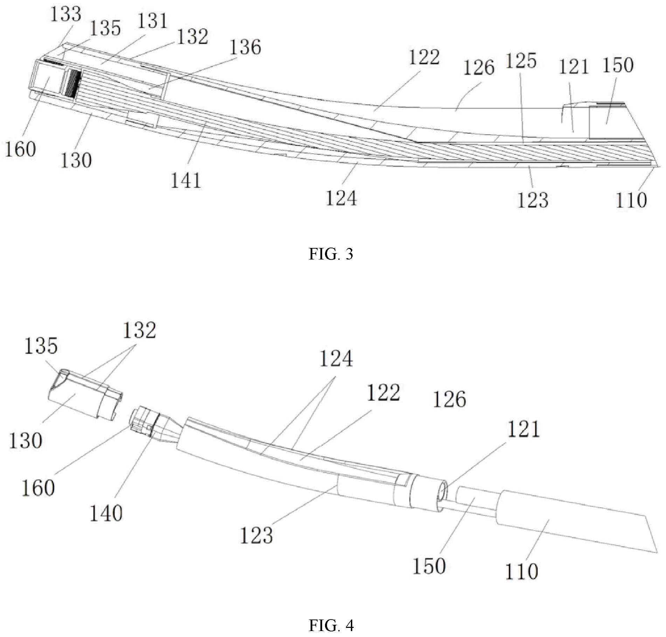

Mounting holes for the imaging module 140 and the illuminating module 160 are designed on the tip segment 130, and the remaining space is designed as the fourth channel 131. In one embodiment, the imaging module 140 forms an imaging port on the distal end surface of the tip segment 130 and can image a target position, and the illuminating module 160 can provide illumination. The tip segment 130 may be made of a transparent or translucent material. When a transparent material is used, the imaging module 140 may also be assembled in a manner of being completely wrapped inside the tip segment 130. Referring to FIG. 3, in a specific embodiment, a fifth channel 136 arranged side by side with the fourth channel 131 in the radial direction may be arranged on the tip segment 130, and the imaging module 140 is embedded in the fifth channel 136 and located at a distal end of the fifth channel 136. In some other embodiments, the imaging module 140 may also have a size close to that of the fifth channel 136, or the imaging module 140 may be surrounded on all sides by a main body of the tip segment 130. In addition, it should be noted that the imaging module 140 may be coaxially arranged with the tip segment 130, or may be offset toward one radial side of the tip segment 130. In one embodiment, the imaging module 140 is offset toward one radial side and is arranged side by side with the fourth channel 131.

In one embodiment, a distal end of the second channel 121 is a groove structure. The groove structure forms the instrument outlet 126, and a groove wall of the groove structure forms a guiding slope surface 122. The guiding slope surface 122 is configured to guide an instrument to extend radially outward from the insertion tube assembly 100 and prevent the instrument from swinging toward either side in a width direction of the groove structure. The groove structure at the distal end of the second channel 121 is actually also a limiting structure. After the instrument passes out through the instrument channel outlet, the limiting structure can still cause the instrument to reach an imaging region of the endoscope along a specified path, preventing the instrument from accidentally slipping out of the groove structure during rotation and thus injuring internal tissue or causing a medical accident.

To facilitate manufacturing of the open-port segment 120, the second channel 121 and the third channel 125 have a common partition layer, and the guiding slope surface 122 is arranged on the partition layer. Meanwhile, this also enables the second channel 121 and the third channel 125 to have larger radial dimensions.

On the basis of the partition layer, the fourth channel 131 is offset toward one radial side of the tip segment 130, the offset direction is consistent with the direction in which the instrument outlet 126 faces, and the proximal opening of the fourth channel 131 faces a distal side of the partition layer. This facilitates reducing resistance and allows liquid to flow more smoothly from or from the proximal end of the fourth channel 131.

To better guide the instrument and prevent the instrument from slipping off, the groove structure includes a straight portion 123 and an arcuate portion 124. The straight portion 123 is located on a proximal side of the instrument outlet 126 and extends in an axial direction of the open-port segment 120, and the arcuate portion 124 is connected to a distal end of the straight portion 123. When the instrument is rotated, the straight portion 123 corresponding to the instrument outlet 126 can provide a larger deformation space and reduce force on the distal end of the instrument, thereby helping prevent the instrument from slipping out of the groove structure.

In addition, the guiding groove 132 is arranged on the outer peripheral surface of the tip segment 130. The guiding groove 132 is butted with a distal end of the groove structure surface, and a groove wall of the guiding groove 132 forms an extended guiding surface configured to guide bending of the instrument. The guiding groove 132 on the tip segment 130 can guide the instrument over a greater length, which is more beneficial to control of the instrument. Meanwhile, preferably, the guiding groove 132 penetrates through the tip segment 130, thereby not only improving the guiding effect, but also allowing the instrument to be closer to an axis of the insertion tube assembly 100, which is more beneficial to rotation of the instrument.

The handle assembly 200 includes a handle housing 201, an insertion tube mounting seat 202, a sealing adapter 203, an instrument access seal cap 205, an O-ring 206, a one-way duckbill valve 207, and a one-way silicone pad 208. The fluid inflow/outflow assembly 300 mainly includes the infusion pipe 310 and the reflux pipe 320. A proximal end of the main-body segment 110 of the insertion tube assembly 100 is fixedly and hermetically connected to an inner hole at a front end of the insertion tube mounting seat 202. The sealing adapter 203 is inserted into a proximal end of the insertion tube mounting seat 202 and is sealed with the insertion tube mounting seat 202 by two O-rings 206. An annular groove 204 is arranged on the sealing adapter 203 between the two O-rings 206 to form a reflux cavity. A radial through hole is arranged on a bottom wall of the annular groove 204, so that the reflux cavity can communicate with the reflux port 210. The reflux pipe 320 communicates with the reflux cavity through the reflux port 210. A radial through hole may also be arranged on an outer periphery of the instrument channel tube 150, so that the instrument channel tube 150 communicates with the reflux cavity. A distal end of the sealing adapter 203 and together define an irrigation cavity, and the infusion pipe 310 communicates with the irrigation cavity through the inflow port 209, thereby enabling irrigation liquid circulation. An inner hole for mounting the one-way duckbill valve 207 and the one-way silicone pad 208 is designed at a rear end of the sealing adapter 203. The instrument access seal cap 205 is then mounted at the rear end of the sealing adapter 203, and presses the one-way duckbill valve 207 and the one-way silicone pad 208 so that they tightly fit with a step of the sealing adapter 203. The one-way duckbill valve 207 is a one-way valve structure. When no instrument is inserted into the endoscope, the valve can prevent liquid in the instrument channel tube 150 from flowing backward to the instrument access seal cap 205. When an instrument is inserted into the endoscope, the one-way duckbill valve 207 is forced open, and an outer diameter of a handle of the instrument tightly fits with an inner hole of the one-way silicone pad 208, thereby preventing liquid in the instrument channel tube 150 from flowing backward to the instrument access seal cap 205. Thus, whether or not an instrument is inserted into the endoscope, liquid in the instrument channel tube 150 can be prevented from flowing backward to the instrument access seal cap 205, and liquid can be discharged only through the reflux cavity and the reflux pipe 320 to the human body and the endoscope. The other end of the connecting wire 141 has a plug that can be quickly plugged into an image processor of the endoscope, thereby implementing signal transmission and control between the imaging module 140 and the illuminating module 160, the endoscope image processor.

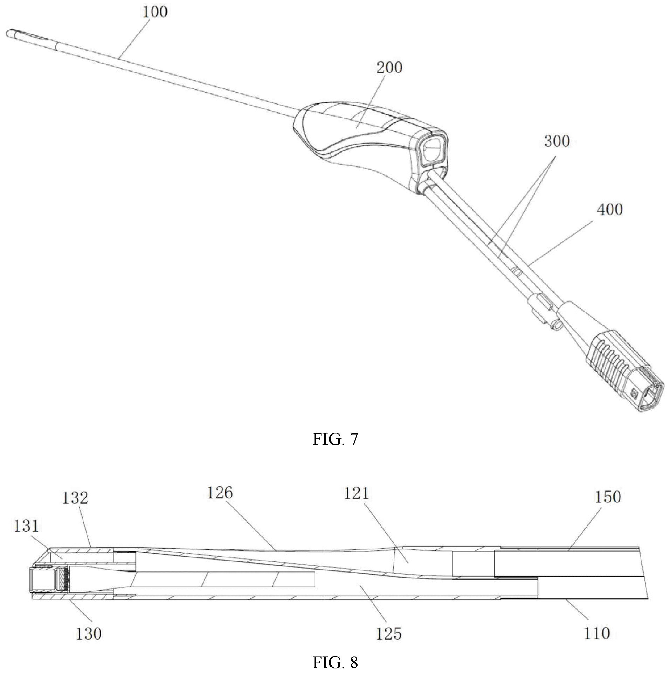

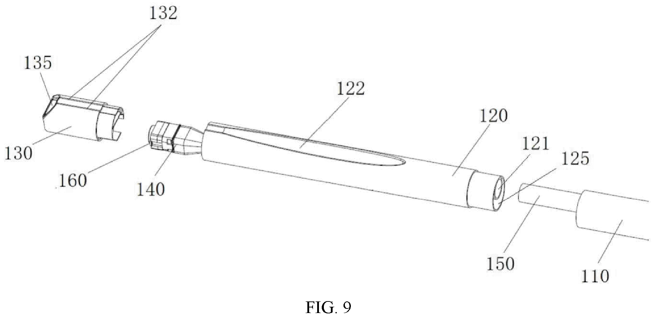

In the above embodiment, the open-port segment 120 of the insertion tube assembly 100 is a curved structure. In some other embodiments, referring to FIG. 7 to FIG. 9, the open-port segment 120 of the insertion tube assembly 100 may also be a straight structure.

During use, the second channel 121 can serve as an instrument channel, so that the instrument channel does not pass through the inside of the tip segment 130. On the premise of ensuring the same inner diameter of the instrument channel, the outer diameter of the insertion tube assembly 100 can be significantly reduced. Meanwhile, the second channel 121 can also serve as a water inlet/reflux channel, and the fourth channel 131, the third channel 125, and the first channel can cooperate to serve as a reflux/water inlet channel. A flow cross section of the channels is large, irrigation efficiency is higher, and a distance between an inflow opening and a reflux opening can be increased, so that liquid circulation is better formed, patient pain is reduced, and doctor operation is facilitated.

An embodiment of the insertion tube assembly of the endoscope of the present utility model is provided below.

The structure of the insertion tube assembly for the endoscope is the same as the structure of the insertion tube assembly 100 in any one of the foregoing embodiments of the endoscope, and details are not described herein again.

The present utility model has been described above by using specific examples. The examples are merely used to help understand the present utility model, and are not intended to limit the present utility model. A person skilled in the art to which the present utility model pertains may make several simple deductions, variations, or replacements according to the concept of the present utility model.

## Claims

1. An insertion tube assembly for an endoscope, comprising:
a main-body segment configured to be connected to a distal end of a handle assembly of the endoscope, a first channel being arranged in the main-body segment;
an open-port segment connected to a distal end of the main-body segment, a second channel and a third channel being arranged in the open-port segment, the second channel and the third channel being isolated from each other, at least a portion of the second channel and the third channel being arranged side by side in a radial direction of the open-port segment, an instrument outlet communicating with the second channel being arranged on an outer peripheral surface of the open-port segment, and the third channel communicating with the first channel;
a tip segment connected to a distal end of the open-port segment, a fourth channel being arranged in the tip segment, a through-flow port communicating with the fourth channel being arranged on a distal end surface of the tip segment, and a proximal end of the fourth channel communicating with the third channel;
an imaging module mounted on the tip segment; and
an instrument channel tube arranged in the first channel, a distal end of the instrument channel tube being hermetically connected to a proximal end of the second channel, and a proximal end of the instrument channel tube being configured to be connected to the handle assembly of the endoscope.

2. The insertion tube assembly for the endoscope according to claim 1, wherein a distal end of the second channel is a groove structure, the groove structure forms the instrument outlet, a groove wall of the groove structure forms a guiding slope surface, the guiding slope surface is configured to guide an instrument out of the insertion tube assembly and prevent the instrument from swinging toward either side in a width direction of the groove structure, the second channel and the third channel have a common partition layer, and the guiding slope surface is arranged on the partition layer.

3. The insertion tube assembly for the endoscope according to claim 2, wherein the fourth channel is offset toward one radial side of the tip segment, an offset direction of the fourth channel is consistent with a direction in which the instrument outlet faces, and a proximal opening of the fourth channel faces a distal side of the partition layer.

4. The insertion tube assembly for the endoscope according to claim 2, wherein the groove structure comprises a straight portion and an arcuate portion, the straight portion is located on a proximal side of the instrument outlet and extends in an axial direction of the open-port segment, and the arcuate portion is connected to a distal end of the straight portion.

5. The insertion tube assembly for the endoscope according to any one of claims 2 to 4, wherein a guiding groove is arranged on an outer peripheral surface of the tip segment, the guiding groove is butted with a distal end of the groove structure surface, and a groove wall of the guiding groove forms an extended guiding surface configured to guide bending of the instrument.

6. The insertion tube assembly for the endoscope according to claim 5, wherein the guiding groove penetrates through the tip segment.

7. The insertion tube assembly for the endoscope according to any one of claims 1 to 4, wherein a cross section of the second channel on a side close to the third channel is arcuate, thereby causing wedge-shaped spaces to be formed at two circumferential ends of the third channel.

8. The insertion tube assembly for the endoscope according to any one of claims 1 to 4, wherein the distal end surface of the tip segment has an inclined portion, the inclined portion is located on at least one circumferential side of the distal end surface, and at least a portion of the through-flow port is located on the inclined portion.

9. The insertion tube assembly for the endoscope according to any one of claims 1 to 4, wherein the fourth channel is located on one radial side of the tip segment, the imaging module is located on another radial side of the tip segment, the fourth channel has a cross section substantially in a shape of an isosceles trapezoid, a first base of the isosceles trapezoid is close to the imaging module, and a second base of the isosceles trapezoid is away from the imaging module.

10. The insertion tube assembly for the endoscope according to claim 9, wherein two ends of the first base have recesses, and the recesses are recessed toward a side of the tip segment on which the imaging module is arranged.

11. The insertion tube assembly for the endoscope according to claim 9, further comprising an illuminating module, wherein the illuminating module is arranged on two sides of the imaging module along an extension direction of a base of the isosceles trapezoid.

12. The insertion tube assembly for the endoscope according to any one of claims 1 to 4, wherein the main-body segment, the open-port segment, and an end-port segment are three components connected in sequence, and the open-port segment is manufactured by injection molding, powder metallurgy, ceramic sintering, or 3D printing.

13. The insertion tube assembly for the endoscope according to any one of claims 1 to 4, wherein the imaging module is connected to a connecting wire, the connecting wire passes through the third channel and the first channel, and a gap for liquid flow is present between the connecting wire and inner walls of the third channel and the first channel.

14. An endoscope, comprising:
the insertion tube assembly according to any one of claims 1 to 13; and
a handle assembly, a proximal end of the insertion tube assembly being connected to the handle assembly, an inflow port and a reflux port being arranged on the handle assembly, one of the inflow port and the reflux port communicating with the proximal end of the instrument channel tube, and the other communicating with a space in the first channel around the instrument channel tube.
